(19) **Europäisches Patentamt European Patent Office Office européen des brevets**

(11) **EP 4 273 547 B1**

(12) **EUROPEAN PATENT SPECIFICATION**

(45) Date of publication and mention of the grant of the patent:
**29.04.2026 Bulletin 2026/18**

(21) Application number: **21912457.5**

(22) Date of filing: **14.01.2021**

(51) International Patent Classification (IPC):
**G01N 21/64** (2006.01)    **C12Q 1/686** (2018.01)

(52) Cooperative Patent Classification (CPC):
**G01N 21/645; C12Q 1/686; G01N 2021/6419;**
**G01N 2021/6421; G01N 2021/6484; Y02A 50/30**

(86) International application number:
**PCT/CN2021/071820**

(87) International publication number:
**WO 2022/141674 (07.07.2022 Gazette 2022/27)**

(54) **PCR DETECTOR AND METHOD THEREFOR**

PCR-DETEKTOR UND VERFAHREN DAFÜR

DÉTECTEUR PAR PCR ET PROCÉDÉ ASSOCIÉ

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **30.12.2020 CN 202011643279**

(43) Date of publication of application:
**08.11.2023 Bulletin 2023/45**

(73) Proprietor: **Integrated Biosystems Co., Ltd.**
**Beijing 100176 (CN)**

(72) Inventors:
• **REN, Lufeng**
  **Beijing 100176 (CN)**
• **JIANG, Pengchong**
  **Beijing 100176 (CN)**
• **FAN, Dongyu**
  **Beijing 100176 (CN)**
• **CAI, Yimei**
  **Beijing 100176 (CN)**
• **GAO, Jing**
  **Beijing 100176 (CN)**

(74) Representative: **Zaboliene, Reda**
**Metida**
**Business center Vertas**
**Gyneju str. 16**
**01109 Vilnius (LT)**

(56) References cited:
EP-A1- 3 334 533       CN-A- 104 662 174
CN-A- 104 677 870    CN-A- 104 677 870
CN-A- 104 792 722    CN-A- 110 619 927
CN-A- 111 157 497    CN-U- 209 836 152
US-A1- 2008 182 264  US-A1- 2009 059 222
US-A1- 2016 061 731

**Description**

The present application requires the priority of a Chinese Patent Application No. 202011643279.7, application name "A PCR detector and its method" submitted to the Chinese Patent Office on December 30, 2020, and the priority of a Chinese Patent Application No. 202023334358.8, application name "A PCR detector" submitted to the Chinese Patent Office on December 30, 2020.

**TECHNICAL FIELD**

The present disclosure relates to the PCR detection field, in particular to a PCR detector and a method thereof.

**BACKGROUND**

A real-time fluorescent quantitative PCR technology refers to a method of performing a quantitative analysis on an unknown template by adding fluorophore in a PCR reaction system, using fluorescence signal accumulation to monitor the whole PCR process in real time and finally using a standard curve.

An important concept - Ct value is in the fluorescent quantitative PCR technology. C represents Cycle, t represents threshold, and the meaning of the Ct value is the cycle number of a fluorescence signal in each reaction tube when reaching a setting threshold.

The patent literature No. CN107923922A discloses an improved sub-component used in a diagnostic test system suitable for receiving a test box and a control method thereof. The sub-component includes an optical detection/excitation device. Wherein an excitation block 910 includes an LED light source 911, which leads light to pass through an optical filter and a lens 912, and then pass through a rod-like lens 913, thereby emitting light with an expected wavelength to an expected position of a reaction container 33. An optical detection block 920 includes a photodiode detector 921, which detects the light emitted from the reaction container 33. Before being received by the photodiode detector 921, the emitted light passes through the rod-like lens 923, the optical filter and the lens 922, thereby ensuring to detect a specific wavelength. The specific wavelength may indicate a reaction for a target analyte in the reaction container 33. At the same time, an optical excitation component 910 and an optical detection component 920 are positioned on an optical mounting member suitable for receiving the plane reaction container 33. The optical excitation component 910 is positioned to emit excitation energy through an edge (a minor face) of a flat surface of the reaction container 33, and the optical detection component 920 is positioned along a main flat surface of the reaction container. In one aspect, the optical excitation component and the optical detection component are orthogonal to each other. A fluorescence point-collecting principle is a conventional algorithm (filtering band-pass of the optical filter). As shown in FIG. 7, the patent literature No. CN111157497A discloses an excitation light source for a hand-held detector, including a PCB board, light emitting lamp banks, an optical filter, an optical fiber coupler, an optical fiber, an optical fiber pump combiner, an optical fiber collimator, a shell and a base plate, wherein the light with different wavelengths, emitted by a plurality of light emitting lamp banks arranged in parallel, enters the corresponding optical fiber through the optical fiber coupler after denoising through the optical filter, is mixed in the optical fiber pump combiner, and finally performs fluorescence excitation on a target through the optical fiber collimator; and each light emitting lamp bank is provided with the optical filter.

In short, in order to detect a target analyte in the prior art, a filter component will be used for filtering treatment after using an excitation light source and a reflected light source, and the photodiode detector is used for detection. After filtering treatment, the emitting light enters the detector for detection, only one emitting light can be detected, and a plurality of emitting light cannot be detected at the same time. In addition, during the detection, fluorescent detection substances may be unevenly distributed in a reaction bin, even not distributed in the reaction bin.

CN104677870A discloses a Superminiaturization multi-channel real-time fluorescent spectrum detector.

US2009/059222A1 discloses a fully integrated microfluidic system.

EP3334533A1 discloses a multiplexed detection of nucleic acid targets directly from samples containing blood.

US2008/182264A1 discloses a systems and methods for baseline correction using non-linear normalization.

US2016/061731A1 discloses an analysis method and system for analyzing a nucleic acid amplification reaction.

**SUMMARY**

In order to solve the above problems in the prior art, the present disclosure provides a PCR detector and a method thereof, an optical filter cannot be used for excitation light and emitting light, and a spectrograph is adopted to detect the emitting light, so as to detect the emitting light with different wavelengths at the same time.

The specific technical solution provided by the present disclosure is as follows:

On the one hand, the present disclosure provides a PCR detector, including an excitation light source module, a chip device and a detection part; and the excitation light source module sends excitation light with a preset wavelength to a chip device, and when performing an amplified reaction, a detection part is arranged at one side of a reaction bin of the chip device;

the chip device includes the reaction bin, in which a detected sample can be contained, wherein the detected sample is a nucleic acid fragment solution including a fluorescence mark;

the excitation light source module is configured to emit the excitation light, and the excitation light emitted by the excitation light source module can illuminate the detected sample arranged in the reaction bin;

the excitation light source module can emit the excitation light of at least two different frequency bands at the same time;

the reaction bin is arranged in an emitting direction of the excitation light of the excitation light module, the detection part is arranged at one side of the reaction bin, and the emitting light formed after the excitation light illuminates the reaction bin can be detected by the detection part; the emitting direction of the excitation light is located below the detection part, and the detection part is configured to detect the emitting light emitted by the detected sample in a vertical direction due to the illumination of the excitation light;

the detection part includes a spectrograph, a wavelength scope of a spectrum detected by the spectrograph is 340-850nm, and the spectrograph can detect the excitation light and the emitting light.

The spectrograph detects the frequency band and intensity of the excitation light of at least two different frequency bands, and the spectrograph detects the frequency band and intensity of the excitation light of at least two different frequency bands and the emitting light caused by the excitation light illuminating the detection sample.

The frequency band of the excitation light of at least two different frequency bands at least includes a first excitation light frequency band and a second excitation light frequency band, and the frequency band of the emitting light caused by the excitation light of two different frequency bands at least includes a first emitting light frequency band and a second emitting light frequency band; and no mutually overlapped frequency band scope is between every two of the first excitation light frequency band, the second excitation light frequency band, the first emitting light frequency band and the second emitting light frequency band.

No mutually overlapped frequency band scope is between the excitation light frequency bands of the excitation light of at least two different frequency bands, and no mutually overlapped frequency band scope is between the different emitting light frequency bands of the emitting light caused by the excitation light of at least two different frequency bands; and no mutually overlapped frequency band scope is between each excitation light frequency band and each emitting light frequency band caused by the excitation light.

The excitation light source module includes a plurality of light emitting units, and each light emitting unit is a light emitting diode.

The excitation light source module includes a PCB board, an optical fiber pump combiner, a plurality of light emitting diodes and at least one optical fiber corresponding to each light emitting diode;

the light emitting diode is arranged at one side of the PCB board, the optical fiber coupler is arranged at an output end of each light emitting diode, each optical fiber coupler is respectively coupled to one optical fiber, and the excitation light with the corresponding wavelength is coupled to the optical fiber through the optical fiber coupler and transmitted through the optical fiber;

the optical fiber pump combiner arranges and combines various optical fibers integrally according to a preset method, and the optical fiber collimator is also arranged at an output end of the optical fiber pump combiner, so as to transform the excitation light in the optical fiber into collimating light;

at least one light emitting diode is configured to emit the excitation light of at least two different frequency bands, and no mutually overlapped frequency scope is in the frequency scope of the excitation light of at least two different frequency bands.

Preferably, the chip device includes a sample adding layer and a pipeline layer, which are arranged in turn from top to bottom. The sampling adding layer includes a sample adding hole and a reagent tube, the sample adding hole is configured to add a sample, and the reagent tube is configured to convey a buffer solution. The pipeline layer includes a reaction bin, in which a freeze-dried reagent is embedded, the freeze-dried reagent includes a fluorescence mark substance, and after being mixed, the sample and the buffer solution in the sample adding hole enter the reaction bin, so as to obtain a detected sample.

Preferably, the fluorescence mark substance is at least two of fam, hex, cy5 and cy5.5, wherein the detected samples of the cy5 and cy5.5 fluorescence marks cannot be detected at the same time, and the detected samples of the fam and hex fluorescence marks cannot also be detected at the same time.

The excitation light source module further includes a light source rotating device, which enables the PCB board with a plurality of light emitting units to rotate, namely, the light source rotating device is arranged on the PCB board in the present disclosure, so that light spots of the emitting light caused by the excitation light are uniformly distributed in the reaction bin, and the detection part can detect more accurately.

Each light emitting unit is an LED light source.

Preferably, the fluorescence mark of the detected sample is fam, hex ,cy5 and cy5.5, when the light emitting unit is the LD light source, the detected sample of the fam and cy 5.5 fluorescence marks can be detected at the same time.

On the other hand, the present disclosure provides a PCR detection method, including the steps of:

collecting cycle 0-40 fluorescence intensity $A_i A_i$, $i \in [0, 40]$ through the spectrograph.

Preferably, further including performing normalization processing on the collected cycle 0-40 fluorescence intensity $A_i$, to obtain $D_i = C_i - y_i$, $i \in [1, 40]$.

Preferably, the normalization processing method for the collected cycle 0-40 fluorescence intensity $A_i$ specifically includes the steps of:

deducting a substrate: taking $A_1 \sim A_{40}$ as a signal, and subtracting the substrate $A_0$;

$$B_i = A_i - A_0, i \in [1, 40]$$

smoothing data: performing smoothing treatment on the data; $B_i$ *smoothdata:'sgolay',9*

$$\xrightarrow{smoothdata:'sgolay',9} C_i$$

confirming a baseline: selecting $C_3 \sim C_{10}$, and fitting a straight line as the baseline according to the least square method; and

$$y_i = ax_i + b, i \in [0, 40]$$

deducting the baseline from the data, to obtain $D_i = C_i - y_i$, $i \in [1, 40]$.

Preferably, the method further includes the treatment of a first order difference, a second order difference and a third order difference for $D_i$ obtained after the normalization processing, as follows:

the first order difference;

$$D_i' = \frac{D_{i+1} - D_{i-1}}{2}, i \in [2, 39]$$

the second order difference;

$$D_i'' = \frac{D_{i+2}' + D_{i-2}' - 2D_i'}{4}, i \in [4, 37]$$

the third order difference;

$$D_i''' = D_{i+1}'' - D_i'', i \in [4, 36]$$

Preferably, the method further includes taking a maximum value of the second order difference, specifically: selecting a maximum point conforming to the law of "positive-negative-negative-negative"→"positive-negative-negative"→"positive-negative" from the third order difference, the first order difference of the corresponding cycle is required to be greater than 0, this coordinate +1 is the maximum value corresponding coordinate of the second order difference, and three points are provided for ±1.

Preferably, the method further includes fitting of a polynomial, fitting the above three points according to a quadratic function ($y = ax^2 + bx + c$), and taking $X = -\frac{b}{2a}$

Preferably, the method further includes solving a Ct value;

drawing a tangent line of the $D_i$ fitted curve beside a X position, wherein an abscissa of an intersection point of the tangent line and the baseline is the Ct value, and then the threshold is obtained;

$$\text{threshold} = (\lceil Ct \rceil - Ct)C_{\lfloor Ct \rfloor} + (Ct - \lfloor Ct \rfloor)C_{\lceil Ct \rceil}$$

Preferably, the method further includes negative/positive judgment:
if the Ct value is $\geq 38$, the Ct value is negative; and otherwise, the Ct value is positive.

Preferably, the method further includes drawing:
drawing a curve for $D_i$ according to the cycle number.

Compared with the prior art, the present disclosure has the following beneficial effects:

(1) Without using a filter, the spectrograph adopted for the detector provided by the present disclosure simplifies the structure composition and reduces the parts;
(2) The spectrograph provided by the present disclosure may detect different multi-channel spectrum, thereby realizing detecting a plurality of fluorescence marks at the same time, namely, detecting various different nucleic acids at the same time, so that the detection efficiency is improved, and the limitation for only detecting one emitting light once by the filter lens is broken. Moreover, the direction of the emitting light provided by the present disclosure is vertical to that of the detection part, so as to avoid the phenomenon of explosion caused by the strong excitation light.
(3) The detection method provided by the present disclosure, for example, the method for solving the Ct value, ensures the simple method and high accuracy, and the negative and positive judgment has high reliability.
(4) The excitation light module provided by the present disclosure is provided with the light source rotating device, which can drive the light emitting unit on the PCB board to rotate, so that the light spots of the emitting light are uniformly distributed.

**BRIEF DESCRIPTION OF THE DRAWINGS**

FIG. 1 is a fluorescence spectrum obtained through detection of a detection part in the present disclosure.
FIG. 2 is a comparison diagram of a fluorescence value and an emitting peak after and before amplification of a single channel fam provided by the present disclosure.
FIG. 3 is a cycle schematic diagram of a fluorescence value and a temperature of a fam channel provided by the present disclosure.
FIG. 4 is a curve of an emitting peak fluorescence value taken according to cycle of a fam channel provided by the present disclosure.
FIG. 5 is a structure diagram of a light source-a chip-a spectrograph provided by the present disclosure.
FIG. 6 is an optical path diagram of a light source-a chip-a spectrograph provided by the present disclosure.
FIG. 7 is a structural schematic diagram of an excitation light source module provided in the prior art.
FIG. 8 is a structural schematic diagram of a chip device provided by the present disclosure.
FIG. 9 is a structural schematic diagram of an overall sample adding layer of an excitation light source module provided by the present disclosure.
FIG. 10 is a structural schematic diagram of a pipeline layer of an overall sample adding layer of an excitation light source module provided by the present disclosure.
FIG. 11 is a Ct value obtained by one embodiment of the present disclosure.
FIG. 12 is distribution of fluorescence mark substances provided by the present disclosure on light spots of emitting light.
FIG. 13 is distribution of fluorescence mark substances on light spots of emitting light after adjusting a light source rotating device provided by the present disclosure.

**DETAILED DESCRIPTION OF THE EMBODIMENTS**

A PCR detector and a method for the PCR detector provided by the present disclosure are specifically described below in combination with drawings.

The present disclosure provides a PCR detector, including an excitation light source module, a chip device and a detection part; and the excitation light source module sends excitation light with a preset wavelength to the chip device, and when performing an amplified reaction, the detection part is arranged above a reaction bin of the chip device;

the chip device includes a reaction bin, in which a detected sample can be contained, wherein the detected sample is a nucleic acid fragment solution including a fluorescence mark;
the excitation light source module is configured to emit the excitation light, and the excitation light emitted by the excitation light source module can illuminate the detected sample arranged in the reaction bin;

the excitation light source module can emit the excitation light of at least two different frequency bands at the same time;

the reaction bin is arranged in an emitting direction of the excitation light of the excitation light module, the detection part is arranged at one side of the reaction bin, and the emitting light formed after the excitation light illuminates the reaction bin can be detected by the detection part; the emitting direction of the excitation light is a horizontal direction, the emitting direction of the excitation light is vertical to the detection part, and the detection part is configured to detect the emitting light emitted by the detected sample in a vertical direction due to the illumination of the excitation light;

the detection part provided by the present disclosure includes a spectrograph, a wavelength scope of a spectrum detected by the spectrograph is 340-850nm, and the spectrograph can detect the excitation light and the emitting light.

The spectrograph detects the frequency band and intensity of the excitation light of at least two different frequency bands, and the spectrograph detects the frequency band and intensity of the excitation light of at least two different frequency bands and the caused emitting light.

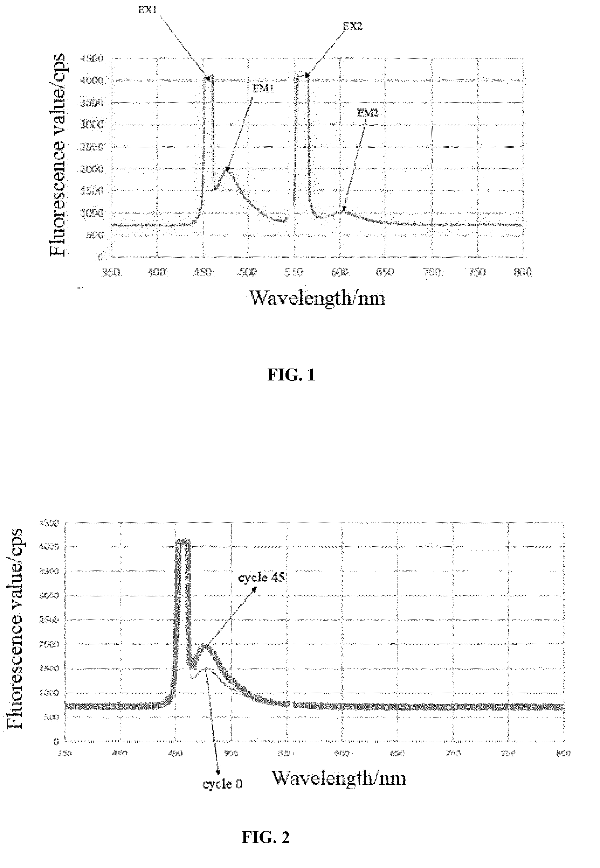

As shown in FIG. 1, which is a result schematic diagram for detecting Fam and cy5.5 channels of the fluorescence mark substance at the same time through the spectrograph provided by the present disclosure, wherein EX1 is FIG. 1 is a peak value of the fam excitation light, EM1 is a peak value of the fam emitting light, EX1 is a peak value of the cy5.5 excitation light, and EM1 is a peak value of the cy5.5 emitting light; and the excitation light of two different frequency bands includes a first excitation light frequency band and a second excitation light frequency band, and the first excitation light frequency band, the second excitation light frequency band, the first emitting light frequency band and the second emitting light frequency band are not mutually overlapped or partially overlapped. Namely, the spectrograph can detect the fam excitation light, the fam emitting light, the cy5.5 excitation light and the cy5.5 emitting light at the same time, wherein the fluorescence mark substance may be fam, hex, cy5, cy5.5 and the like, as shown in the table below, and the light source is an LD light source. When the light source is the LD light source, the detected samples of the fam and cy5.5 fluorescence marks can be detected at the same time, the detected samples of the cy5 and cy5.5 fluorescence marks cannot be detected at the same time, and the detected samples of the fam and hex fluorescence marks cannot also be detected at the same time.

For the detected samples of the fam and cy5.5 fluorescence marks, the excitation wavelength of the fam fluorescence mark is 494nm, the emitting wavelength is 518nm, the fluorescent wavelength of the cy5.5 fluorescence mark is 675nm, and the emitting wavelength is 695nm, namely, a completely overlapped frequency band is not available among the excitation wavelength and the emitting wavelength of the fam fluorescence mark, and the excitation wavelength and the emitting wavelength of the cy5.5 fluorescence mark.

For example, the first frequency band scope of the excitation light is 450-480, however the second frequency band scope of the excitation light is 540-560nm, namely the first frequency band scope of the excitation light and the second frequency band scope of the excitation light are the frequency band scopes without overlapping.

The first frequency band scope of the excitation light is 520-550, however the second frequency band scope of the excitation light is 540-560nm, namely the first frequency band scope of the excitation light and the second frequency band scope of the excitation light are the frequency band scopes without partial overlapping.

The list for the wavelength of the excitation light source provided by the present disclosure as the LD light source, the excitation wavelength and the emitting wavelength of the fluorescence mark substances fam, hex, cy5 and cy5.5 is as shown in table 1 below.

Table 1 is the list for the wavelength of the LD light source, the excitation wavelength and the emitting wavelength of the fluorescence mark substances fam, hex, cy5 and cy5.5.

| LD wavelength (nm) | Maximum light power (mW) | | Probe dye | Excitation wavelength (nm) | Emitting wavelength (nm) |
|---|---|---|---|---|---|
| 405 | 55 | | fam | 494 | 518 |
| 485 | 85 | | hex | 520 | 548 |
| 520 | 80 | | Cy5 | 649 | 670 |
| 638 | 30 | | Cy5.5 | 675 | 695 |

As a preferred implementation mode, the spectrograph detects the frequency band and intensity of the excitation light of at least two different frequency bands, and the spectrograph detects the frequency band and intensity of the excitation light of at least two different frequency bands and the emitting light caused by the excitation light illuminating the detection sample.

The frequency band of the excitation light of at least two different frequency bands at least includes a first excitation light frequency band and a second excitation light frequency band, and the frequency band of the emitting light caused by the excitation light of two different frequency bands at least includes a first emitting light frequency band and a second emitting light frequency band; and no mutually overlapped frequency band scope is between every two of the first excitation light frequency band, the second excitation light frequency band, the first emitting light frequency band and the second emitting light frequency band.

No mutually overlapped frequency band scope is between the excitation light frequency bands of the excitation light of at least two different frequency bands, and no mutually overlapped frequency band scope is between the different emitting light frequency bands of the emitting light caused by the excitation light of at least two different frequency bands; and no mutually overlapped frequency band scope is between each excitation light frequency band and each emitting light frequency band caused by the excitation light.

As a preferred implementation mode, the chip device provided by the present disclosure includes a sample adding layer and a pipeline layer, which are arranged in turn from top to bottom. The sampling adding layer includes a sample adding hole and a reagent tube, the sample adding hole is configured to add a sample, and the reagent tube is configured to convey a buffer solution. The pipeline layer includes a reaction bin, in which a freeze-dried reagent is embedded, the freeze-dried reagent includes a fluorescence mark substance, and after being mixed, the sample and the buffer solution in the sample adding hole enter the reaction bin, so as to obtain a detected sample. The buffer solution is a buffer solution, which refers to a mixed solution composed of weak acid and its salt, weak base and its salt, so as to cancel and alleviate the impact of the added strong acid or strong base on the solution PH to some extent, thereby maintaining a relatively stable pH value of the solution.

As a preferred implementation mode, the excitation light source module provided by the present disclosure further includes a light source rotating device, which enables the PCB board with a plurality of light emitting units to rotate, namely, the light source rotating device may be arranged on the PCB board in the present disclosure, so that light spots of the emitting light caused by the excitation light are uniformly distributed in the reaction bin, and the detection part can detect more accurately.

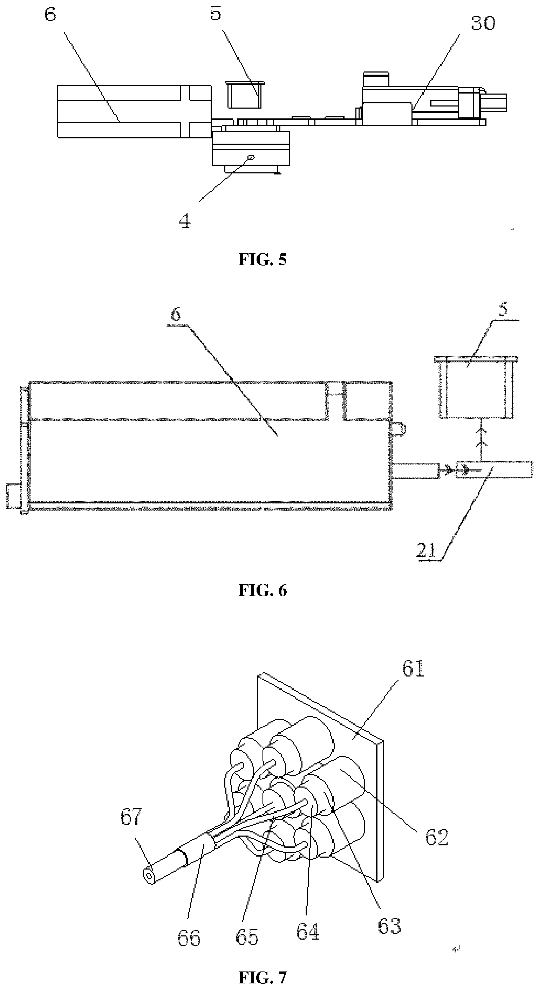

Referring to FIG. 5, which is an optical path schematic diagram of a light source-a chip-a spectrograph provided by the present disclosure. The system of this embodiment includes a chip device 30, an excitation light module 6, a temperature control module 4 and a detection part 5, the excitation light module 6 sends out excitation light with a preset wavelength to the chip device, after the reaction, fluorescent information is acquired through the fluorescent detection part 5 to complete the detection, and the temperature control module 4 controls the temperature in the chip device, so that a reagent in the chip device can reach the best reaction state. When an amplified reaction is carried out in this embodiment, the detection part 5 is arranged above the reaction bin of the chip device.

Referring to FIG. 6, the device applied in the emitting light detection method provided by this embodiment of the present disclosure includes an excitation light source module 6, a detected sample 21 and a detection part 5, the light emitting unit illuminates the detected sample 21 to excite the detected sample 21, and the detection part 5 detects the emitting light generated by the detected sample; the detected sample 21 includes fluorophore, which is used to generate the emitting light under the excitation of the excitation light; the detection part is arranged on the optical path of the emitting light, so as to catch and detect the emitting light; and specifically, FIG. 6 shows an optical path diagram, and the optical path of the excitation light and the optical path of the emitting light are shown by different arrows, so as to show the difference of two light.

Specifically, those skilled in the art can understand that the function of the detection part 6 is to catch and detect the emitting light. During an actual application process, the spectrograph may be a fluorescence spectrograph, which allows reading a plurality of emitting light and excitation light at the same time, thereby performing detection. The spectrograph provided by the present disclosure does not include an optical filter, so the excitation light of a plurality of different frequency bands and the emitting light caused by the excitation light can be detected at the same time. For example, the spectrograph may be Hamamatsu C12889MA.

The excitation light source module includes a plurality of light emitting units, and each light emitting unit is a light emitting diode.

The excitation light source module includes a PCB board, an optical fiber pump combiner, at least one light emitting diode and at least one optical fiber corresponding to each light emitting diode;

the light emitting diode is arranged at one side of the PCB board, an optical fiber coupler is arranged at an output end of each light emitting diode, each optical fiber coupler is respectively coupled to one optical fiber, and the excitation light with the corresponding wavelength is coupled to the optical fiber through the optical fiber coupler and transmitted through the optical fiber;
the optical fiber pump combiner arranges and combines various optical fibers integrally according to a preset method, and an optical fiber collimator is also arranged at an output end of the optical fiber pump combiner, so as to transform the excitation light in the optical fiber into collimating light;

at least one light emitting diode is configured to emit the excitation light of at least two different frequency bands, and no mutually overlapped frequency scope is in the frequency scope of the excitation light of at least two different frequency bands.

Specifically, as shown in FIG. 7, the difference between the present disclosure and the excitation light source module in the prior art is that the excitation light source module provided by the present disclosure does not include an optical filter 63, and the excitation light source module in the prior art must include the optical filter 63; specifically, as shown in FIG. 7, the optical excitation component provided by the present disclosure includes an excitation light source module, the excitation light module 6 adopts a light emitting module with a smaller volume, and the excitation light in the excitation light source module is switched by adopting a switch, thereby generating excitation light with different wavelengths. The excitation light module 6 includes a PCB board 61, which is configured to load the excitation light source, such as an LED light source or an LD light source or other light sources, and the LED light source is arranged at one side of the PCB board. In this embodiment, the LED light source includes a plurality of arranged LED lamps 62, an optical fiber coupler 64 is arranged at the front end of each LED lamp, each optical fiber coupler 64 is respectively coupled to an optical fiber 65, the excitation light with the corresponding wavelength is coupled to the optical fiber through the optical fiber coupler 64 and transmitted through the optical fiber; and the excitation light module 6 further includes an optical fiber pump combiner 66, which arranges and combines various optical fibers integrally according to a preset method, and an optical fiber collimator 67 is also arranged at an output end of the optical fiber pump combiner 66, so as to transform the excitation light in the optical fiber into collimation excitation light.

The excitation light source module provided by the present disclosure further includes a light source rotating device, which enables the PCB board with a plurality of light emitting units to rotate, namely, the light source rotating device may enable the PCB board to rotate, so as to drive the light emitting unit, namely, the LED light source or the LD light source to rotate, namely, the light source rotating device may be arranged on the PCB board in the present disclosure, so that light spots of the emitting light caused by the excitation light are uniformly distributed in the reaction bin, and the detection part can detect more accurately.

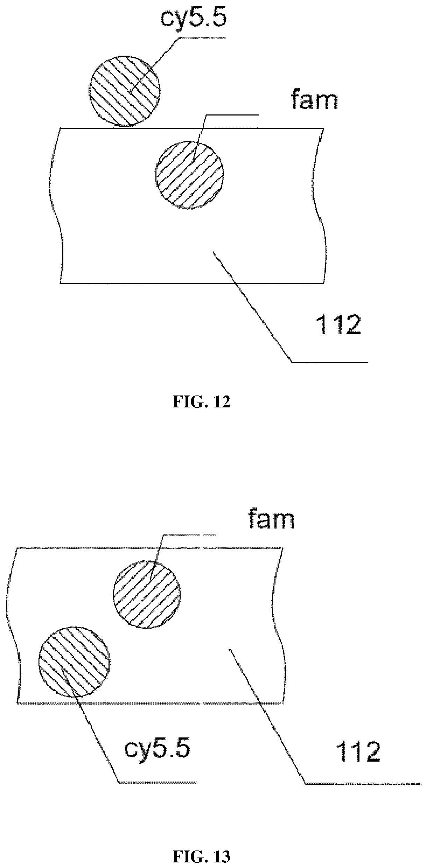

As shown in FIG. 12-FIG. 13, FIG. 12 is the distribution of the fluorescence mark substance on the reaction bin 112 provided by the present disclosure when the excitation light source module does not rotate, namely, is not adjusted, that is, the distribution is uneven, even not distributed on the reaction bin, or the fluorescence mark substance is not distributed on the reaction bin, as shown in FIG. 12. FIG. 13 is the distribution of the fluorescence mark substance on the reaction bin 112 provided by the present disclosure after the light source rotating device is adjusted. After the light source rotating device is adjusted, the fluorescence mark substance is uniformly distributed in the reaction bin 112 and distributed in the same reaction bin, so as to solve the technical problem of uneven distribution in the prior art, as shown in FIG. 13.

The working process of the excitation light source module is as follows: start the LED light 62, send the LED light source, lead the excitation light source with the corresponding wavelength to the optical fiber through the optical fiber coupler, collect a plurality of optical fibers through the optical fiber pump combiner 66, combine the delivered excitation light source as one beam, and finally transform the transmission light into the collimation excitation light through the optical fiber collimator to illuminate a target for detection, wherein a plurality of LED lamps are provided, and can send the excitation light with the different wavelengths at the same time without requiring the optical filter, and the spectrograph used in the present disclosure can identify the emitting light with different wavelengths, so the optical filter is not required.

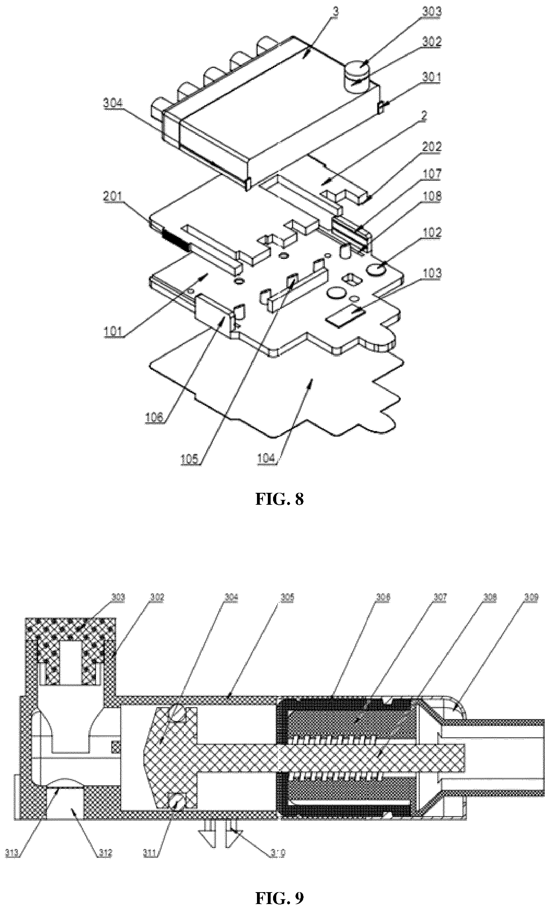

Referring to FIG. 8, the reactor provided by the present disclosure is a chip device, specifically, the chip device of the present disclosure includes a sample adding layer 3 arranged at the uppermost end, a gasket 2 arranged at a lower side of the sample adding layer 3, a pipeline layer 101 arranged at a lower side of the gasket 2 and a sealing film 104 arranged at the lowermost layer, wherein a sample adding hole 302 is formed in the upper side of the sample adding layer 3, so as to add a sample in the chip, and the sample injected into the chip must undergo the extracting, purification and amplified reaction. In this embodiment, the sample adding layer and the pipeline layer are movably connected to a limiting frame 106 at the side of the pipeline layer 101 through a card slot 304, correspondingly, a first neck 107 is arranged in the limiting frame 106, and the first neck realizes switching and fixing the sampling adding layer and the pipeline layer at a relative position through the mutual matching and connection with the card slot. The sealing film is adhered to the lower side of the pipeline layer 101, so as to realize sealing.

A second neck 109 is also arranged at the side of the limiting frame on the lower side of the first neck 107, correspondingly, a second card slot (not shown in the figure) is also arranged on a side wall of the gasket 2, the mutual matching and connection of the second card slot and the second neck can realize the sliding connection between the gasket 2 and the pipeline layer 101, and the switching and fixing of the relative position can be realized through the second cart slot and the second neck. In this embodiment of the present disclosure, a first sliding rail 202 is also arranged at the lower side of the gasket 2, correspondingly, a second sliding groove 108 is arranged at the upper side of the pipeline layer 101, the matched connection between the first sliding rail 202 and the second sliding groove 108 can realize the sliding connection between the gasket and the pipeline layer 101, and the switching and fixing at the relative position can be realized through the first sliding rail 202 and the second sliding groove 108. In this embodiment, the second sliding groove 108 is arranged in the limiting frame 106 on the pipeline layer. A plurality of notches and bulges alternately arranged are arranged at the end of the gasket 2, wherein the first sliding rail 202 is arranged on a bottom surface of the bulge at the outermost side.

Referring to FIG. 8, in the present disclosure, a sample adding hole cover 303 is arranged on the sample adding hole for sealing. Buckle structures are also arranged on the sample adding layer and the pipeline layer, a first buckle 301 is arranged at one side of the sample adding layer, a stretching end at the lower side of the first buckle 301 stretches out of a bottom end of the sample adding layer, and after being matched and installed together, the sample adding layer and the pipeline layer are buckled on the side of the pipeline layer through the first buckle, thereby preventing the separation of the sample adding layer and the pipeline layer. In this embodiment, two first single valves 102 are arranged on the pipeline layer so as to inject a sample and a reaction reagent into the pipeline respectively; double valves 103 are also arranged on the pipeline layer so as to inject the reagent and the sample; and the double valves 1033 communicate with the reaction bin 112 through the pipeline. As shown in FIG. 5, handles 201 are also arranged at the two sides of the gasket 2, so as to extract the chip device conveniently. In this embodiment of the present disclosure, the reaction bin is arranged at the edge of the pipeline layer, the reaction bin is a semi-elliptic structure, so the reaction reagent can react, and the convenient positioning and installation can be realized by a raised semi-elliptic structure during use.

Referring to FIG. 8, in the present disclosure, a row of spikes 105 are arranged on the pipeline layer, after extracting the gasket, the spikes communicate with the reagent in the sample adding layer, and when a fluorescent sequence marked in the reagent is in complementary matching with a nucleic acid spike at a corresponding position, a group of spike sequence with completely complemented sequence is obtained by confirming a position of a probe with the strongest fluorescence intensity. A baffle is also arranged outside the spikes, and when the sample adding layer matches with the pipeline layer, the baffle plays a blocking and positioning role.

Specifically speaking, in this embodiment of the present disclosure, a plurality of groups of reagent tubes are arranged in the sample adding layer during a sample adding state, the sample adding layer 3 is buckled with the first neck 107 through the card slot 107 on the sample adding layer 3. During an initial installation state, the sample adding layer 3 matches with the pipeline layer 101 from top to bottom, and the spikes are isolated from the reagent in the reagent tube through the gasket 2, thereby preventing mixing the spikes with the reagent caused by vibration during transportation, and avoiding puncturing. When a test is required, the gasket 2 is drawn out along the second sliding groove 108. After the gasket 2 is drawn out along the second sliding groove, the sample adding layer 3 is pressed downwards, so that the card slot 304 on the sample adding layer is buckled with the second neck 109, at this time, the spikes arranged on the pipeline layer are mixed with the reagent of the sample adding layer, and then the reagent is introduced to the pipeline layer for determination.

Specifically speaking, in the present disclosure, the chip device can store the reagent by setting the gasket structure, so the reagent is completely stored during the transportation, and when the reagent is used, the gasket is drawn out, namely, the reagent can be introduced into the pipeline layer.

Referring to FIG. 9, which is a structural schematic diagram of a sample adding layer of one embodiment provided by the present disclosure. A sample adding bin is arranged below the sample adding hole 302 in this embodiment, the sample adding bin can be connected to a reagent tube loading the reagent, a reagent outlet 312 is arranged at the lower part of the sample adding bin, and a sealing structure is arranged between the reagent outlet 312 and the sampling bin for sealing. A pressurized structure is also arranged at one side of the sample adding bin, the pressurized structure includes a tube wall 305, in which a piston 308 is arranged, the piston 308 moves to the sample adding bin so as to push the reagent in the sample adding bin to flow out of the reagent outlet; and a sealing ring 311 is arranged at the end of a piston rod of the piston 308 for sealing.

Referring to FIG. 9, in this embodiment, a nut 307 is also arranged on the piston rod, and a relative rotational motion is realized through a threaded connection with the nut 307. Correspondingly, one end of the piston rod is provided with an output structure, such as a gas cylinder or an oil cylinder, or the piston rod may be connected by rotating the output structure, such as a motor and a screw. At this time, the piston rod is in the rotational motion, and only pushes the reagent to flow out of the reagent outlet. Correspondingly, a guide sleeve 306 is sleeved outside the nut, a corresponding shaft shoulder is arranged in the tube wall for positioning and fixing the guide sleeve 306; and snap rings 314 are also arranged outside the two ends of the guide sleeve 306 so as to lock the corresponding guide sleeve 306. A jacket 309 is also arranged outside the guide sleeve 306, so as to protect the piston rod, the nut and the guide sleeve. When the pipeline layer is subjected to the reagent injection, the pressure in the pipeline layer is increased by moving the piston to the sample adding bin, so as to push the reagent to flow out of the reagent outlet and realize the reagent injection. However, during an actual application process, in order to match with the reagent injection and improve the reagent injection efficiency, the high-efficiency reagent injection may be realized through outward absorption of the reagent tubes and matching with the inward pushing of the current reagent tube. In this embodiment of the present disclosure, a plurality of groups of reagent tubes are arranged. In this embodiment, five groups of reagent tubes are arranged to apply reagent to the pipeline layer, and the reagent may be lysate, eluant, cleaning fluid and the like, which can improve the use efficiency greatly.

Referring to FIG. 9, a second buckle 310 is arranged below the sample adding layer, and the second buckle is arranged at one side relative to the fist buckle, so as to prevent the sample adding layer from sliding.

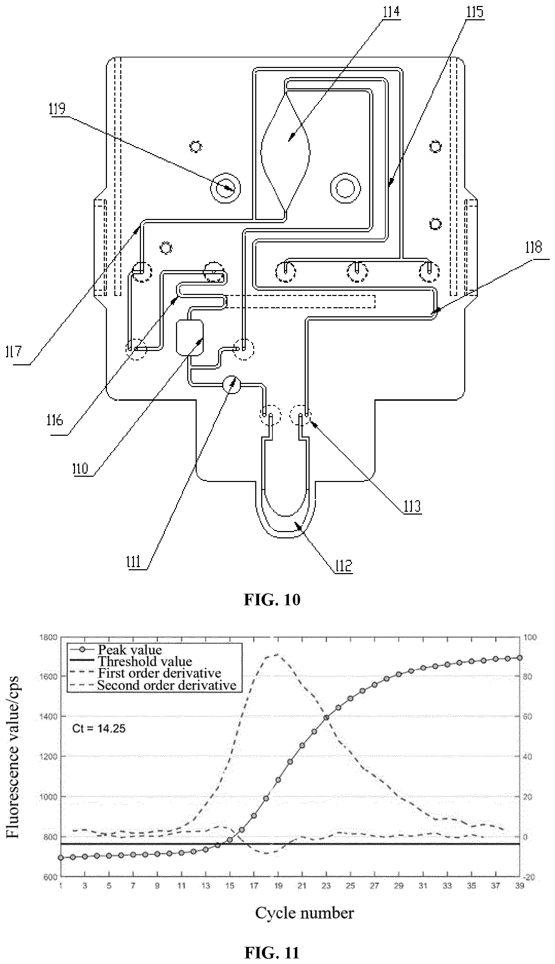

Referring to FIG. 10, which is a structural schematic diagram of a pipeline layer of one embodiment provided by the present disclosure. In this embodiment, the pipeline layer is provided with a reaction bin 112, a first buffer bin 110, a second buffer bin 111 and a purification bin 114 for purifying a sample, wherein first ends of the double valves are connected to the purification bin 114 through a first pipeline 118 and a second pipeline 115; second ends of the double valves communicate with the second buffer bin 111, the second buffer bin 111 is connected to the first buffer bin 110 through a pipeline, a pipeline branch is also arranged on the pipeline between the first buffer bin and the second buffer bin, a second single valve is arranged on the pipeline branch, the other end of the pipeline branch is connected to the purification bin 114, and the first buffer bin is connected to the purification bin 114 through a third pipeline 116 and the fourth pipeline 117; and a plurality of connecting holes 119 are formed in the pipeline layer for connecting.

Specifically speaking, the first pipeline 118 includes a vertical pipeline and a transverse pipeline, a nucleic acid substance enters an amplification bin through long-distance transportation and elution, the second pipeline 115 includes a vertical pipeline and a transverse pipeline, and one end of the second pipeline 115 is connected to the purification bin while the other end is integrally connected to the first pipeline.

Specifically speaking, a third pipeline 116 is a multi-way bending pipe, of which one end is connected to the first buffer bin while the other end is connected to a first liquid inlet; and one end of the fourth pipeline 117 is connected to a first liquid inlet while the other end is connected to the purification bin, and a simple inlet and a first single valve are also arranged on the fourth pipeline 117.

Specifically speaking, the simple inlet is configured to add a sample, a first liquid inlet is configured to add lysate, and then the first single valve is opened, so that the sample and the lysate are subjected to a mixed reaction. During a mixing process, pushing and absorption operations may be performed by using the piston rod connected to a sample port and the piston rod connected to a first liquid inlet, thereby realizing the sufficient mixing of the sample and the lysate and generating a first reactant. The first reactant is liquid, the liquid enters the purification bin through a fourth pipeline 117, a magnetic bead is arranged in the purification bin, which is a reaction bin for extracting and purifying the nucleic acid, and then the first single valve is closed and the second single valve is opened. A second reagent is injected into a second liquid inlet, the second reagent is a cleaning fluid, and the second reagent enters into the purification bin through the pipeline connected to the second reagent port so as to clean the substance in the purification bin. A third reagent is injected into the third liquid inlet, the third reagent is a cleaning fluid, the third reagent enters into the purification bin through the pipeline connected to the third reagent, and then the nucleic acid substance in the purification bin is cleaned again. A fourth reagent is injected into a fourth liquid inlet, the fourth reagent is eluant, the fourth reagent enters the purification bin through the pipeline connected to the fourth reagent, and the nucleic acid substance in the purification bin is washed from the magnetic bead arranged on the nucleic acid substance, so as to obtain the nucleic acid substance. The nucleic acid substance is introduced into the reaction bin 112 through the first pipeline 115 and the second pipeline 118 for an amplified reaction. In the present disclosure, when the pipeline layer is subjected to reagent injection, the pressure in the pipeline layer is increased by moving the piston to the sample adding bin, so as to push the reagent to flow out of the reagent outlet and realize the reagent or sample injection. A plurality groups of piston structures are arranged in the present disclosure, and the sample or reagent is regularly applied to the pipeline layer, so as to improve the use efficiency greatly. The amplification bin is arranged at the edge of the pipeline layer, the reaction bin 112 is a semi-elliptic structure, so the reaction reagent can react, the convenient positioning and installation during use can be realized by the raised semi-elliptic structure; and the chip device is transparent.

Specifically, the gasket and the related connecting structure provided by the present disclosure, on the one hand, enable the sample adding layer to completely connect to the pipeline layer, thereby avoiding vibration, and on the other hand, enable the spikes to have a better placement space. The gasket and the sample adding layer are in sliding installation for convenient disassembly. During an initial installation state, the sample adding layer matches with the pipeline layer from top to bottom, and the spikes are isolated from the reagent in the reagent tube through the gasket, thereby preventing mixing the spikes with the reagent caused by vibration during transportation, and avoiding puncturing. When a test is required, the gasket is drawn out along the second sliding groove. After the gasket is drawn out along the second sliding groove, the sample adding layer is pressed downwards, so that the card slot on the sample adding layer is buckled with the second neck, at this time, the spikes arranged on the pipeline layer are mixed with the reagent of the sample adding layer, and then the reagent is introduced to the optical path layer for determination. In the present disclosure, the chip device can store the reagent by setting the gasket structure, so the reagent is completely stored during the transportation, and when the reagent is used, the gasket is drawn out, namely, the reagent can be introduced into the pipeline layer.

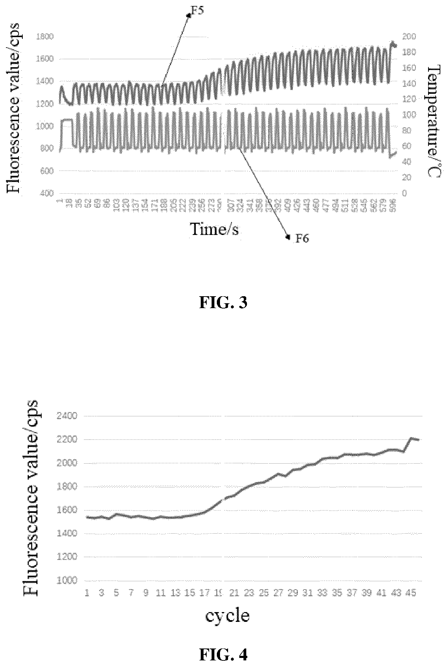

As shown in FIG. 2, which is a comparison diagram of a fluorescence value and an emitting peak after and before amplification of a single channel fam provided by the present disclosure, wherein Cycle 0 is a non-amplified fluorescence spectrum curve, and cycle 45 is a fluorescence spectrum curve after amplifying 45 cycles. As shown in FIG. 3, which is a cycle schematic diagram of a fluorescence value and a temperature of a fam channel provided by the present disclosure, wherein the abscissa is the time, the ordinate is the fluorescence value at the emitting peak of the fam channel, thus the variation trend of the fluorescence curve along the temperature can be seen. F5 is a variation curve of the fluorescence value along the time, F6 is a variation curve of the temperature along the time, FIG. 3 leads out the curve of FIG. 4 taking the fluorescence value according to cycle, FIG.4 is a curve of an emitting peak fluorescence value taken according to cycle of a fam channel provided by the present disclosure. Within 3s before the end of an extension phase of a PCR amplification process, a lighting time point may perform a data processing flow for a PCR detection method after obtaining a curve.

Specifically, a PCR detection method includes the steps of:
an original data:

collecting cycle 0-40 fluorescence intensity through a Hamamatsu micro spectrograph;

$$A_i, i \in [0, 40]$$

deducting a substrate;
taking $A_1 \sim A_{40}$ as a signal, and subtracting the substrate $A_0$;

$$B_i = A_i - A_0, i \in [1, 40]$$

smoothing data;

performing smoothing treatment on the data based on matlab self-function smoothdata (parameters method: sgolay, winsize: 9);

$$B_i \xrightarrow{smoothdata:'sgolay',9} C_i$$

or removing substrate data $B_i$ for smoothing treatment by Savitzky-Golay smoothing algorithm function sg_smooth, wherein the parameter is winsize:3, and the degree is 6;
$B_i \rightarrow C_i$
confirming a baseline:

selecting $C_3 \sim C_{10}$ (optional index), and fitting a straight line as the baseline according to a least square method; and

$$y_i = ax_i + b, i \in [0, 40]$$

normalizing;

deducting the baseline from the data;

$$D_i = C_i - y_i, i \in [1, 40]$$

the first order difference;

$$D_i' = \frac{D_{i+1} - D_{i-1}}{2}, i \in [2, 39]$$

the second order difference;

$$D_i'' = \frac{D_{i+2}' + D_{i-2}' - 2D_i'}{4}, i \in [4, 37]$$

the third order difference; and

$$D_i''' = D_{i+1}'' - D_i'', i \in [4, 36]$$

taking a maximum value of the second order difference (the maximum value of the second order difference can be obtained by calculating the third order difference).

specifically, selecting a maximum point conforming to the law of "positive-negative-negative-negative"or"-positive-negative-negative"or"positive-negative" from the third order difference (to find the maximum point from the second order difference, the front, middle and rear points of the third order difference corresponding to the point may be compared), the first order difference of the corresponding cycle is required to be greater than 0 (screening the three laws in turn, if it is screened in the front law, the next law is not performed, otherwise, the screening is performed again), this coordinate +1 is the maximum value corresponding coordinate of the second order difference, and three points are provided for the coordinate $\pm 1$ corresponding to the maximum value of the two order difference;

fitting of a polynomial

fitting the above points according to a quadratic function *(y = ax² + bx + c),* and taking

$$X = -\frac{b}{2a}$$

a tangent line for solving a Ct value;

drawing the tangent line of the $D_i$ fitted curve beside the X position, wherein an abscissa of an intersection point of the tangent line and the baseline is the Ct value, and then the threshold is obtained;

$$threshold = (\lceil Ct \rceil - Ct)C_{\lfloor ct \rfloor} + (Ct - \lfloor Ct \rfloor)C_{\lceil Ct \rceil}$$

negative/positive judgment:

if the Ct value is $\geq 38$, the Ct value is negative; and otherwise, the Ct value is positive;

drawing;

drawing a curve as shown in FIG. 11 for $D_i$ according to the cycle number.

The above is only preferred embodiments of the present disclosure and is not intended to limit the present disclosure. Those skilled in the art may make various modifications and variations. Any modifications, equivalent replacements, improvements and the like made within the scope of the appended claims shall fall within the scope of protection of the present disclosure.

## Claims

1.  A PCR detector, comprising an excitation light source module, a chip device (30) and a detection part (5); and the excitation light source module sends excitation light with a preset wavelength to the chip device (30), and when performing an amplified reaction, the detection part (5) is arranged at one side of a reaction bin of the chip device (30);

    the chip device (30) comprises the reaction bin, in which a detected sample (21) is contained, wherein the detected sample (21) is a nucleic acid fragment solution including a fluorescence mark;
    the excitation light source module is configured to emit the excitation light, and the excitation light emitted by the excitation light source module is capable of illuminating the detected sample (21) arranged in the reaction bin;
    the excitation light source module is capable of emitting the excitation light of at least two different frequency bands at the same time;
    wherein the reaction bin is arranged in an emitting direction of the excitation light of the excitation light module (6), the detection part (5) is arranged at one side of the reaction bin, and the emitting light formed after the excitation light illuminates the reaction bin is capable of being detected by the detection part (5); the emitting direction of the excitation light is located below the detection part (5), and the detection part (5) is configured to detect the emitting light emitted by the detected sample (21) in a vertical direction due to the illumination of the excitation light; and the detection part (5) comprises a spectrograph, a wavelength scope of a spectrum detected by the spectrograph is 340-850nm, and the spectrograph is capable of detecting the excitation light and the emitting light;

the excitation light source module includes a PCB board, an optical fiber pump combiner, a plurality of light emitting diodes and at least one optical fiber corresponding to each light emitting diode;

the light emitting diodes are arranged at one side of the PCB board, the optical fiber coupler is arranged at an output end of each light emitting diode, each optical fiber coupler is respectively coupled to one optical fiber, and the excitation light with the corresponding wavelength is coupled to the optical fiber through the optical fiber coupler and transmitted through the optical fiber;

the optical fiber pump combiner arranges and combines various optical fibers integrally according to a preset method, and the optical fiber collimator is also arranged at an output end of the optical fiber pump combiner, so as to transform the excitation light in the optical fiber into collimating light;

at least one light emitting diode is configured to emit the excitation light of at least two different frequency bands, and no mutually overlapped frequency scope is in the frequency scope of the excitation light of at least two different frequency bands;

the excitation light source module further includes a light source rotating device, which is configured to rotate the PCB board with the plurality of light emitting diodes.

2. The PCR detector according to claim 1, wherein the spectrograph detects the frequency band and intensity of the excitation light of at least two different frequency bands, and the spectrograph detects the frequency band and intensity of the excitation light of at least two different frequency bands and the emitting light caused by the excitation light illuminating a detection sample.

3. The PCR detector according to claim 2, wherein the frequency band of the excitation light of at least two different frequency bands at least comprises a first excitation light frequency band and a second excitation light frequency band, and the frequency band of the emitting light caused by the excitation light of two different frequency bands at least comprises a first emitting light frequency band and a second emitting light frequency band; and no mutually overlapped frequency band scope is between every two of the first excitation light frequency band, the second excitation light frequency band, the first emitting light frequency band and the second emitting light frequency band.

4. The PCR detector according to claim 1, wherein the chip device (30) includes a sample adding layer and a pipeline layer, which are arranged in turn from top to bottom. The sampling adding layer includes a sample adding hole and a reagent tube, the sample adding hole is configured to add a sample, and the reagent tube is configured to convey a buffer solution. The pipeline layer includes a reaction bin, in which a freeze-dried reagent is embedded, the freeze-dried reagent includes a fluorescence mark substance, and after being mixed, the sample and the buffer solution in the sample adding hole enter the reaction bin, so as to obtain the detected sample (21).

5. The PCR detector according to claim 1, wherein the fluorescence mark substance is at least two of fam, hex, cy5 and cy5.5, wherein the detected samples (21) of the cy5 and cy5.5 fluorescence marks cannot be detected at the same time, and the detected samples (21) of the fam and hex fluorescence marks cannot also be detected at the same time.

6. A PCR detection method using the PCR detector of claim 1, comprising the steps of:

S1: collecting cycle 0-40 fluorescence intensity $A_i$, $A_i$, $i \in [0, 40]$ through a spectrograph;
S2: performing normalization processing on the collected cycle 0-40 fluorescence intensity A, to obtain normalized data $D_i$, $D_i = C_i - y_i$, $i \in [1, 40]$, $y_i$ is a baseline, and $C_i$ is a data after smoothing treatment;
S3: performing a first order difference, a second order difference and a third order difference for $D_i$ after the normalization processing;
S4: taking a maximum value of the second order difference, selecting a maximum point conforming to the law of "positive-negative-negative-negative" or "positive-negative-negative" or "positive-negative" from the third order difference, the first order difference of the corresponding cycle is required to be greater than 0, this coordinate +1 is the maximum value corresponding coordinate of the second order difference, and three points are provided for $\pm 1$;
S5: fitting of a polynomial, fitting the above three points obtained in S4 according to a quadratic function ($y = ax^2 + bx + c$), and taking

$$X = -\frac{b}{2a},$$

wherein a is a quadratic coefficient, b is a primary coefficient, and c is a constant;

S6: solving a Ct value through a tangent line, drawing the tangent line of the $D_i$ fitted curve beside a X position, an abscissa of an intersection point of the tangent line and the baseline is the Ct value, and then the threshold is obtained;

$$threshold = (\lceil Ct \rceil - Ct)C_{\lfloor Ct \rfloor} + (Ct - \lfloor Ct \rfloor)C_{\lceil Ct \rceil};$$

S6: negative/positive judgement: and

if the Ct value is $\geq$ 38, the Ct value is negative; and otherwise, the Ct value is positive.

**7.** The PCR detection method according to claim 6, wherein the method further comprises a step of S7: drawing, and drawing a curve for $D_i$ according to the cycle number.

**8.** The PCR detection method according to claim 6, wherein the normalization processing method for the collected cycle 0-40 fluorescence intensity $A_i$ specifically comprises the steps of:

S11: deducting a substrate: taking $A_1$ - $A_{40}$ as a signal, and subtracting the substrate $A_0$;

$$B_i = A_i - A_0, i \in [1, 40],$$

wherein $B_i$ is the data after removing the substrate;

S12: smoothing data: performing smoothing treatment on the data $B_i$ after removing the baseline, so as to obtain the data $C_i$ after the smoothing treatment;

$B_i \rightarrow C_i$

S13: confirming a baseline: selecting $C_3$ - $C_{10}$, and fitting a straight line as the baseline $y_i$ according to a least square method; and

$$y_i = a_1 \chi_i + b_1, \quad i \in [1, 40],$$

wherein $a_1$ is a primary coefficient, and $b_1$ is a constant; and

S14: deducting the baseline from the data, to obtain $D_i = C_i - y_i$, $i \in [1, 40]$.

**9.** The PCR detection method according to claim 8, wherein the removing substrate data $B_i$ is subjected to smoothing treatment by Savitzky-Golay smoothing algorithm function sg_smooth, the parameter is winsize:3, and the degree is 6.

**10.** The PCR detection method according to claim 6, wherein the first order difference, the second order difference and the third order difference are specifically treated as follows:

S31: the first order difference;

$$D_i' = \frac{D_{i+1} - D_{i-1}}{2}, i \in [2, 39],$$

wherein $D_i'$ is the first order difference value of the ith point, $D_{i+1}$ is the (i+1)th point after the normalization processing, and $D_{i-1}$ is the (i-1)th point after the normalization processing,;

S32: the second order difference;

$$D_i'' = \frac{D_{i+2}' + D_{i-2}' - 2D_i'}{4}, i \in [4, 37],$$

wherein $D_i''$ is the second order difference value of the i$^{th}$ point, $D_{i+2}'$ is the value of (i+2)$^{th}$ point in the first order difference, and $D_{i-2}'$ is value of the (i-2)$^{th}$ point in the first order difference;

S33: the third order difference;

$$D_i''' = D_{i+1}'' - D_i'', i \in [4, 36],$$

wherein $D_i'''$ is the third order difference value of the i$^{th}$ point, $D_{i+1}''$ is the value of (i+1)$^{th}$ point in the second order difference.

## Patentansprüche

1.  PCR-Detektor, umfassend ein Anregungslichtquellenmodul, eine Chipvorrichtung (30) und einen Detektionsteil (5); wobei das Anregungslichtquellenmodul Anregungslicht mit einer voreingestellten Wellenlänge an die Chipvorrichtung (30) sendet, und wobei bei der Durchführung einer Amplifikationsreaktion der Detektionsteil (5) an einer Seite einer Reaktionskammer der Chipvorrichtung (30) angeordnet ist;

    wobei die Chipvorrichtung (30) die Reaktionskammer umfasst, in der eine detektierte Probe (21) enthalten ist, wobei die detektierte Probe (21) eine Nukleinsäurefragmentlösung mit einer Fluoreszenzmarkierung ist; wobei das Anregungslichtquellenmodul zum Emittieren des Anregungslichts konfiguriert ist, und wobei das durch das Anregungslichtquellenmodul emittierte Anregungslicht in der Lage ist, die in der Reaktionskammer angeordnete detektierte Probe (21) zu beleuchten; wobei das Anregungslichtquellenmodul in der Lage ist, das Anregungslicht in mindestens zwei unterschiedlichen Frequenzbändern gleichzeitig zu emittieren; wobei die Reaktionskammer in einer Emissionsrichtung des Anregungslichts des Anregungslichtmoduls (6) angeordnet ist, wobei der Detektionsteil (5) an einer Seite der Reaktionskammer angeordnet ist, und wobei das Emissionslicht, das nach dem Beleuchten der Reaktionskammer durch das Anregungslicht gebildet wird, in der Lage ist, durch den Detektionsteil (5) detektiert zu werden; wobei sich die Emissionsrichtung des Anregungslichts unterhalb des Detektionsteils (5) befindet, und wobei der Detektionsteil (5) dazu konfiguriert ist, das Emissionslicht zu detektieren, das von der detektierten Probe (21) in einer vertikalen Richtung aufgrund der Beleuchtung des Anregungslichts emittiert wird; und wobei der Detektionsteil (5) einen Spektrographen umfasst, wobei ein Wellenlängenbereich eines durch den Spektrographen detektierten Spektrums zwischen 340 und 850 nm liegt, und wobei der Spektrograph in der Lage ist, das Anregungslicht und das Emissionslicht zu detektieren; wobei das Anregungslichtquellenmodul eine Leiterplatte, einen Glasfaser-Pumpkombinierer, mehrere Leuchtdioden und mindestens eine Glasfaser, die zu jeder Leuchtdiode korrespondiert, beinhaltet; wobei die Leuchtdioden auf einer Seite der Leiterplatte angeordnet sind, wobei der Glasfaserkoppler an einem Ausgabeende jeder Leuchtdiode angeordnet ist, wobei jeder Glasfaserkoppler jeweils mit einer Glasfaser gekoppelt ist, und wobei das Anregungslicht mit der entsprechenden Wellenlänge durch den Glasfaserkoppler in die Glasfaser eingekoppelt und durch die Glasfaser übertragen wird; wobei der Glasfaser-Pumpkombinierer gemäß einem voreingestellten Verfahren verschiedene Glasfasern integral anordnet und kombiniert, und wobei der Glasfaserkollimator ebenfalls an einem Ausgabeende des Glasfaser-Pumpkombinierers angeordnet ist, um das Anregungslicht in der Glasfaser in kollimierendes Licht umzuwandeln; wobei mindestens eine Leuchtdiode dazu konfiguriert ist, das Anregungslicht in mindestens zwei unterschiedlichen Frequenzbändern zu emittieren, und wobei in dem Frequenzbereich des Anregungslichts von mindestens zwei unterschiedlichen Frequenzbändern keine sich überlappenden Frequenzbereiche vorliegen; wobei das Anregungslichtquellenmodul ferner eine Lichtquellenrotationsvorrichtung beinhaltet, die dazu konfiguriert ist die Leiterplatte mit den mehreren Leuchtdioden zu rotieren.

2.  PCR-Detektor nach Anspruch 1, wobei der Spektrograph das Frequenzband und die Intensität des Anregungslichts in mindestens unterschiedlichen Frequenzbändern detektiert, und wobei der Spektrograph das Frequenzband und die Intensität des Anregungslichts in mindestens unterschiedlichen Frequenzbändern und das Emissionslicht detektiert, das durch das eine Detektionsprobe beleuchtende Anregungslicht verursacht wird.

3.  PCR-Detektor nach Anspruch 2, wobei das Frequenzband des Anregungslichts in mindestens unterschiedlichen Frequenzbändern zumindest ein erstes Anregungslicht-Frequenzband und ein zweites Anregungslicht-Frequenzband umfasst, und wobei das Frequenzband des Emissionslichts, das durch das Anregungslicht in unterschiedlichen Frequenzbändern verursacht wird, zumindest ein erstes Emissionslicht-Frequenzband und ein zweites Emissionslicht-Frequenzband umfasst; und wobei keine sich überlappenden Frequenzbandbereiche zwischen jeweils zwei des ersten Anregungslicht-Frequenzbandes, des zweiten Anregungslicht-Frequenzbandes, des ersten Emissionslicht-Frequenzbandes und des zweiten Emissionslicht-Frequenzbandes vorliegen.

4. PCR-Detektor nach Anspruch 1, wobei die Chipvorrichtung (30) eine Probenzugabeschicht und eine Leitungsschicht beinhaltet, die nacheinander von oben nach unten angeordnet sind. Die Probenzugabeschicht beinhaltet ein Probenzugabeloch und ein Reagenzröhrchen, wobei das Probenzugabeloch dazu konfiguriert ist, eine Probe zuzugeben, und wobei das Reagenzröhrchen dazu konfiguriert ist, eine Pufferlösung zu befördern. Die Leitungsschicht beinhaltet eine Reaktionskammer, in der ein gefriergetrocknetes Reagenz eingebettet ist, wobei das gefriergetrocknete Reagenz eine Fluoreszenzmarkierungssubstanz enthält, und wobei nach dem Mischen die Probe und die Pufferlösung in dem Probenzugabeloch in die Reaktionskammer gelangen, um die detektierte Probe (21) zu erhalten.

5. PCR-Detektor nach Anspruch 1, wobei es sich bei der Fluoreszenzmarkierungssubstanz um mindestens zwei der folgenden Substanzen handelt: fam, hex, cy5 und cy5.5, wobei die detektierten Proben (21) mit der cy5 und cy5.5 Fluoreszenzmarkierungen nicht gleichzeitig detektiert werden können, und die detektierten Proben (21) mit der fam und hex Fluoreszenzmarkierungen ebenfalls nicht gleichzeitig detektiert werden können.

6. PCR-Nachweisverfahren, das den PCR-Detektor nach Anspruch 1 verwendet und die folgenden Schritte umfasst:

S1: Sammeln einer Fluoreszenzintensität in Zyklus 0 bis 40 $A_i$ durch einen Spektrographen, $A_i$, mit $i \in [0, 40]$;
S2: Durchführen einer Normalisierungsverarbeitung an der gesammelten Fluoreszenzintensität in Zyklus 0 bis 40 $A_i$, um normalisierte Daten $D_i$ zu erhalten, wobei $D_i = C_i - y_i$ mit $i \in [1, 40]$ gilt, wobei $y_i$ für eine Basislinie und $C_i$ für ein Datum nach einer Glättungsbehandlung steht;
S3: Durchführen einer Differenz erster Ordnung, einer Differenz zweiter Ordnung und einer Differenz dritter Ordnung für $D_i$ nach der Normalisierungsverarbeitung;
S4: Nehmen eines Maximalwerts der Differenz zweiter Ordnung, Auswählen eines Maximalpunkts, der dem Gesetz von "positiv-negativ-negativ-negativ" oder "positiv-negativ-negativ" oder "positiv-negativ" entspricht, aus der Differenz dritter Ordnung, wobei die Differenz erster Ordnung des entsprechenden Zyklus größer als 0 sein muss, diese Koordinate +1 die dem Maximalwert entsprechende Koordinate der Differenz zweiter Ordnung ist, und drei Punkte für $\pm 1$ bereitgestellt sind;
S5: Anpassen eines Polynoms, Anpassen der obigen in S4 erhaltenen drei Punkte gemäß einer quadratischen Funktion ($y = ax^2 + bx + c$), und Nehmen

$$X = -\frac{b}{2a},$$

wobei a für einen quadratischen Koeffizienten, b für einen primären Koeffizient, und c für eine Konstante steht;
S6: Lösen eines Ct-Werts durch eine Tangente, Zeichnen der Tangente der angepassten $D_i$-Kurve neben einer X-Position, wobei eine Abszisse eines Schnittpunkts der Tangente und der Basislinie der Ct-Wert ist und dann der Schwellenwert erhalten wird;

$$\text{Schwellenwert} = (\lceil Ct \rceil - Ct)C_{\lfloor Ct \rfloor} + (Ct - \lfloor Ct \rfloor)C_{\lceil Ct \rceil};$$

S6: Beurteilung von negativ/positiv: und
wobei bei Ct-Wert $\geq 38$ der Ct-Wert negativ ist und andernfalls der Ct-Wert positiv ist.

7. PCR-Nachweisverfahren nach Anspruch 6, wobei das Verfahren ferner einen Schritt von S7 umfasst: Zeichnen, und Zeichnen einer Kurve für $Di$ gemäß der Zyklusnummer.

8. PCR-Nachweisverfahren nach Anspruch 6, wobei die Normalisierungsverarbeitungsmethode für die gesammelte Fluoreszenzintensität in Zyklus 0 bis 40 Ai spezifisch die folgenden Schritte umfasst:

S11: Abziehen eines Substrats: Nehmen von $A_1 - A_{40}$ als ein Signal, und Subtrahieren des Substrats $A_0$;
wobei $B_i = A_i - A_0$ mit $i \in [1, 40]$ gilt, wobei $B_i$ für die Daten nach dem Entfernen des Substrats steht;

S12: Glätten von Daten: Durchführen einer Glättungsbehandlung an den Daten $B_i$ nach dem Entfernen der Basislinie, um die Daten $C_i$ nach der Glättungsbehandlung zu erhalten;

$B_i \rightarrow C_i$

S13: Bestätigen einer Basislinie: Auswählen von $C_3$ - $C_{10}$ und Anpassen einer geraden Linie als die Basislinie $y_i$ gemäß einer Methode der kleinsten Quadrate; und

wobei $y_i = a_1 X_i + b_1$ mit $i \in [1, 40]$ gilt, wobei $a_1$ für einen primären Koeffizienten und $b_1$ für eine Konstante steht; und

S14: Abziehen der Basislinie von den Daten, um $D_i = C_i - y_i$ zu erhalten, mit $i \in [1, 40]$.

9. PCR-Nachweisverfahren nach Anspruch 8, wobei die nach dem Entfernen des Substrats erhaltenen Daten $B_i$ einer Glättungsbehandlung durch eine Savitzky-Golay-Glättungsalgorithmus-Funktion sg_smooth unterzogen werden, wobei der Parameter winsize:3 ist, und der Grad 6 ist.

10. PCR-Nachweisverfahren nach Anspruch 6, wobei die Differenz erster Ordnung, die Differenz zweiter Ordnung und die Differenz dritter Ordnung spezifisch wie folgt behandelt werden:

S31: die Differenz erster Ordnung;

wobei $D_i' = \frac{D_{i+1} - D_{i-1}}{2}$ mit $i \in [2, 39]$ gilt, wobei $D_i'$ für den Differenzwert erster Ordnung des i-ten Punkts, $D_{i+1}$ für den (i+1)-ten Punkt nach der Normalisierungsverarbeitung, und $D_{i-1}$ für den (i-1)-ten Punkt nach der Normalisierungsverarbeitung steht;

S32: die Differenz zweiter Ordnung;

wobei $D_i'' = \frac{D_{i+2}' + D_{i-2}' - 2D_i'}{4}$ mit $i \in [4, 37]$ gilt, wobei $D_i''$ für den Differenzwert zweiter Ordnung des i-ten Punkts, $D_{i+2}'$ für den Wert des (i+2)-ten Punkts in der Differenz erster Ordnung, und $D_{i-2}'$ für den Wert des (i-2)-ten Punkts in der Differenz erster Ordnung steht;

S33: die Differenz dritter Ordnung;

wobei $D_i''' = \frac{D_{i+1}'' - D_i''}{4}$ mit $i \in [4, 36]$ gilt, wobei $D_i'''$ für den Differenzwert dritter Ordnung des 1-ten Punkts und $D_{i+1}''$ für den Wert des (i+1)-ten Punkts in der Differenz zweiter Ordnung steht.

## Revendications

1. Détecteur PCR, comprenant un module de source de lumière d'excitation, un dispositif à puce (30) et une portion de détection (5) ; le module de source de lumière d'excitation émet une lumière d'excitation à une longueur d'onde prédéterminée vers le dispositif à puce (30), et lorsqu'une réaction amplifiée est effectuée, la portion de détection (5) est disposée à un côté d'une cuve de réaction du dispositif à puce (30) ; le dispositif à puce (30) comprend la cuve de réaction, dans laquelle est placé un échantillon détecté (21), l'échantillon détecté (21) étant une solution de fragment d'acide nucléique comportant un marqueur fluorescent ;

le module de source de lumière d'excitation est configuré pour émettre la lumière d'excitation, et la lumière d'excitation émise par le module de source de lumière d'excitation est capable d'illuminer l'échantillon détecté (21) disposé dans la cuve de réaction ;

le module de source de lumière d'excitation est capable d'émettre la lumière d'excitation d'au moins deux bandes de fréquences différentes en même temps ;

dans lequel la cuve de réaction est disposée dans une direction d'émission de la lumière d'excitation du module de lumière d'excitation (6), la portion de détection (5) est disposée à un côté de la cuve de réaction, et la lumière d'émission formée après que la lumière d'excitation illumine la cuve de réaction est détectée par la portion de détection (5) ; la direction d'émission de la lumière d'excitation est située sous la portion de détection (5), et la portion de détection (5) est configurée pour détecter la lumière d'émission émise par l'échantillon détecté (21) dans une direction verticale en raison de l'illumination par la lumière d'excitation ; et

la portion de détection (5) comprend un spectrographe, une plage de longueur d'onde d'un spectre détecté par le spectrographe est de 340 à 850 nm, et le spectrographe est capable de détecter la lumière d'excitation et la lumière d'émission ;

le module de source de lumière d'excitation comprend une carte PCB, un combinateur de pompe à fibre optique, une pluralité de diodes électroluminescentes et au moins une fibre optique correspondant à chaque diode électroluminescente ;

les diodes électroluminescentes sont disposées à un côté de la carte PCB, un coupleur de fibre optique est disposé à une extrémité de sortie de chaque diode électroluminescente, chaque coupleur de fibre optique est respectivement couplé à une fibre optique, et la lumière d'excitation à longueur d'onde correspondante est couplée à la fibre optique par l'intermédiaire du coupleur de fibre optique et transmise à travers la fibre optique ;

le combinateur de pompe à fibre optique organise et combine diverses fibres optiques de manière intégrale selon un procédé prédéfini, et un collimateur à fibre optique est également disposé à une extrémité de sortie du combinateur de pompe à fibre optique, afin de transformer la lumière d'excitation dans la fibre optique en lumière collimatée;

au moins une diode électroluminescente est configurée pour émettre la lumière d'excitation d'au moins deux bandes de fréquences différentes, et aucune plage de fréquences se chevauchant ne se trouve dans la plage de fréquences de la lumière d'excitation d'au moins deux bandes de fréquences différentes ; et

le module de source de lumière d'excitation comprend en outre un dispositif de rotation de source de lumière, qui est configuré pour faire tourner la carte PCB avec la pluralité de diodes électroluminescentes.

**2.** Détecteur PCR selon la revendication 1, dans lequel le spectrographe détecte la bande de fréquences et l'intensité de la lumière d'excitation d'au moins deux bandes de fréquences différentes, et le spectrographe détecte la bande de fréquences et l'intensité de la lumière d'excitation d'au moins deux bandes de fréquences différentes ainsi que la lumière d'émission causée par la lumière d'excitation illuminant un échantillon de détection.

**3.** Détecteur PCR selon la revendication 2, dans lequel la bande de fréquences de la lumière d'excitation d'au moins deux bandes de fréquence différentes comprend au moins une première bande de fréquences de la lumière d'excitation et une seconde bande de fréquences de la lumière d'excitation, et la bande de fréquences de la lumière d'émission causée par la lumière d'excitation de deux bandes de fréquences différentes comprend au moins une première bande de fréquences de la lumière d'émission et une seconde bande de fréquences de la lumière d'émission ; et aucune plage de bande de fréquences se chevauchant ne se trouve entre chaque deux parmi la première bande de fréquences de la lumière d'excitation, la seconde bande de fréquences de la lumière d'excitation, la première bande de fréquences de la lumière d'émission et la seconde bande de fréquences de la lumière d'émission.

**4.** Détecteur PCR selon la revendication 1, dans lequel le dispositif à puce (30) comporte une couche d'ajout d'échantillon et une couche de tuyauterie, qui sont disposées successivement de haut en bas ; la couche d'ajout d'échantillon comporte un orifice d'ajout d'échantillon et un tuyau de réactif, l'orifice d'ajout d'échantillon étant configuré pour ajouter un échantillon, et le tuyau de réactif étant configuré pour transporter une solution tampon ; la couche de tuyauterie comporte une cuve de réaction, dans laquelle est intégré un réactif lyophilisé, le réactif lyophilisé comporte une substance marqueuse fluorescente, et une fois mélangé, l'échantillon et la solution tampon dans l'orifice d'ajout d'échantillon entre dans la cuve de réaction, afin d'obtenir l'échantillon détecté (21).

**5.** Détecteur PCR selon la revendication 1, dans lequel la substance marqueuse fluorescente est au moins deux des fam, hex, cy5 et cy5,5, les échantillons détectés (21) des marquages fluorescents cy5 et cy5,5 ne pouvant pas être détectés en même temps, et les échantillons détectés (21) des marquages fluorescents fam et hex ne pouvant pas non plus être détectés en même temps.

**6.** Procédé de détection par PCR utilisant le détecteur PCR selon la revendication 1, comprenant les étapes suivantes :

S1 : collecter une intensité de fluorescence $A_i$, des cycles 0-40 à l'aide d'un spectrographe, $A_i$, $i \in [0, 40]$ ;

S2 : effectuer un traitement de normalisation sur l'intensité de fluorescence $A_i$ collectée des cycles 0-40, afin d'obtenir des données normalisées $D_i$, $D_i = C_i - y_i$, $i \in [1, 40]$, $y_i$ est une ligne de base, et $C_i$ est une donnée après un traitement de lissage ;

S3 : effectuer une différence du premier ordre, une différence du deuxième ordre et une différence du troisième ordre sur $D_i$ après le traitement de normalisation ;

S4 : prendre une valeur maximale de la différence du deuxième ordre, sélectionner un point maximal conformément à la loi « positif-négatif-négatif-négatif » ou « positif-négatif-négatif » ou « positif-négatif » à partir de la différence du troisième ordre, la différence du premier ordre du cycle correspondant doit être supérieure à 0, une coordonnée+1 est une coordonnée correspondant à la valeur maximale de la différence du deuxième ordre, et trois points sont prévus pour ±1 ;

S5 : ajuster un polynôme, ajuster les trois points ci-dessus obtenus à l'étape S4 selon une fonction quadratique *(y = ax² + bx + c)*, et prendre $X = -\dfrac{b}{2a}$, où a est un coefficient quadratique, b est un coefficient primaire, et c est une constante ;

S6 : résoudre une valeur $C_t$ par une ligne tangente, tracer la ligne tangente de la courbe ajustée pour $D_i$ à côté d'une position X, une abscisse d'un point d'intersection entre la ligne tangente et la ligne de base étant la valeur $C_t$, puis obtenir le seuil ;

$$\text{seuil} = (\lceil Ct \rceil - Ct)C_{\lfloor Ct \rfloor} + (Ct - \lfloor Ct \rfloor)C_{\lfloor Ct \rfloor};$$

S6 : effectuer un jugement négatif/positif : et
si la valeur Ct est ≥ 38, la valeur Ct est négative ; sinon, la valeur Ct est positive.

**7.** Procédé de détection par PCR selon la revendication 6,
dans lequel le procédé comprend en outre une étape S7 : tracer une courbe pour $D_i$ selon le nombre de cycles.

**8.** Procédé de détection par PCR selon la revendication 6,
dans lequel le procédé de traitement de normalisation pour l'intensité de fluorescence $A_i$ collectée des cycles 0-40 comprend en particulier les étapes suivantes :

S11 : déduire un substrat : prendre $A_1$-$A_{40}$ comme signal, et soustraire le substrat $A_0$ ;

$$B_i = A_i - A_0, i \in [1, 40],$$

où $B_i$ est la donnée après la suppression du substrat ;
S12 : faire lisser des données : effectuer un traitement de lissage sur la donnée $B_i$ après la suppression de la ligne de base, afin d'obtenir les données $C_i$ après le traitement de lissage ;
$B_i \rightarrow C_i$
S13 : confirmer une ligne de base : sélectionner $C_3$-$C_{10}$, et ajuster une ligne droite comme ligne de base $y_i$ selon une méthode des moindres carrés ; et
$y_i = a_1 x_i + b_1$, $i \in [1, 40]$, où al est un coefficient primaire, et $b_1$ est une constante ; et
S14 : déduire la ligne de base de la donnée, afin d'obtenir $D_i = C_i - y_i$, $i E [1, 40]$.

**9.** Procédé de détection par PCR selon la revendication 8,
dans lequel lors de la suppression du substrat, la données $B_i$ est soumises à un traitement de lissage par une fonction d'algorithme sg_smooth de lissage de Savitzky-Golay, le paramètre est winsize : 3, et le degré est de 6.

**10.** Procédé de détection par PCR selon la revendication 6,
dans lequel la différence du premier ordre, la différence du deuxième ordre et la différence du troisième ordre sont traitées en particulier comme suit :

S31 : la différence du premier ordre ;

$$y_i = a_l x_i + b_l,$$

$$D_i' = \frac{D_{i+1} - D_{i-1}}{2}, i \in [2, 39],$$

où $D_i'$ est la valeur de la différence du premier ordre du premier point, $D_{i+1}$ est le (i+1) -ème point après le traitement de normalisation, et $D_{i-1}$ est le (i-1)-ème point après le traitement de normalisation ;
S32 : la différence du deuxième ordre ;

$$D_i'' = \frac{D_{i+2}' + D_{i-2}' - 2D_i'}{4}, i \in [4, 37],$$

où $D_i''$ est la valeur de la différence du troisième ordre du i-ème point, $D_{i+2}'$ est la valeur du (i+2) -ème point dans la différence du deuxième ordre, et $D_{i-2}'$ est la valeur du (i-2) -ème point dans la différence du premier ordre ;

S33 : la différence du troisième ordre ;

$$D_i''' = D_{i+1}'' - D_i'', \quad D_i \in [4, 37],$$

où $D_i'''$ est la valeur de la différence du troisième ordre du i-ème point, $D_{i+1}''$ est la valeur du (i+1) -ème point dans la différence du deuxième ordre.

**FIG. 1**

**FIG. 2**

**FIG. 3**

**FIG. 4**

**FIG. 5**

**FIG. 6**

**FIG. 7**

**FIG. 8**

**FIG. 9**

**FIG. 10**

**FIG. 11**

cy5.5

fam

112

**FIG. 12**

fam

cy5.5

112

**FIG. 13**

**REFERENCES CITED IN THE DESCRIPTION**

**Patent documents cited in the description**

- CN 202011643279
- CN 202023334358
- CN 107923922 A
- CN 111157497 A
- CN 104677870 A
- US 2009059222 A1
- EP 3334533 A1
- US 2008182264 A1
- US 2016061731 A1